# EUROPEAN PATENT APPLICATION

(11) **EP 2 047 840 A1**
(43) Date of publication of application: **15.04.2009**
(21) Application number: 07019441.0
(22) Date of filing: 04.10.2007
(51) Int. Cl.: A61K 8/37, A61K 8/41, A61K 8/73, A61Q 5/12, A61Q 17/04, A61Q 19/00

(54) **Aqueous meta-stable oil-in-water emulsions**

(71) Applicant: Cognis IP Management GmbH, 40589 Düsseldorf (DE)
(72) Inventor: Amela Conesa, Cristina, 08290 Cerdanyola del Vallés (ES); Domingo, Marta, 08024 Barcelona (ES); Beuché, Marc, 91430 Vauhallan (FR)
(74) Representative: Fabry, Bernd

(57) **Abstract**

Suggested are aqueous o/w emulsions with the ability to invert into w/o emulsions by introduction of mechanical energy, comprising
(a) oil components,
(b) w/o emulsifiers,
(c) cationic surfactants,
(d) actives, and
(e) cationic compatible stabilisers.

The emulsions are useful for making cosmetic or pharmaceutical compositions, particularly sprayable sun care emulsions.

## Description

### Object of the invention

The present invention relates to the area of cosmetic and pharmaceutical compositions and more particularly concerns low viscous, meta-stable o/w emulsions which invert into w/o emulsions when applied to skin.

### State of the art

As water in oil (w/o) emulsion systems are close to the skin's hydrolipid film, they are more effective from the dermatological viewpoint. This emulsion type promotes the long lasting moisturizing efficacy by providing an occlusive film and reinforces the active ingredients action into the stratum corneum. In addition, w/o emulsions leave a lipophilic film on the skin surface which ensures high water repellency, which is an important parameter to maintain high UV protection in sun care applications. Nevertheless, tackiness, combined with greasiness and slow spreading are key factors which tend to decrease cosmetic acceptance, thus counteracting w/o emulsion benefits.

European patent application EP 1174180 B1 (Clariant) claims a process for preparing fine emulsions showing a particle size in the range from 0.1 to 10 µm which are characterised in that a w/o pre-emulsion, comprising at least one w/o emulsifier selected from sorbitol esters, polyglycerol esters, sorbitan esters, fatty acid esters and/or dimethiconcopolyols are treated with at least one surfactant in order to invert said w/o fine emulsion into an o/w fine emulsion on condition that the inversion is conducted without increasing the temperature. However, the emulsions thus obtained do not exhibit a sufficient stability, especially when stored at higher temperatures over a couple of days. In addition, the process requires the preparation of an intermediate w/o emulsion which is turned into an o/w emulsion which makes the manufacture time-consuming and little efficient.

Therefore, the complex problem underlying the present invention has been to develop new o/w emulsions exhibiting
o viscosities low enough to be formulated as sprays;
o long lasting moisturising effect;
o high water resistance;
o enhanced UV protection;
o improved active ingredients efficacy;
o stability under normal storage conditions;
o but inverting into a w/o emulsion when applied to skin
while at the same time avoiding the disadvantages known from the state of the art.

### Description of the invention

The present invention claims aqueous meta-stable o/w emulsions with the ability to invert into w/o emulsions by introduction of mechanical energy, comprising
(a) oil components,
(b) w/o emulsifiers,
(c) cationic surfactants,
(d) actives, and
(e) cationic compatible stabilisers.

Surprisingly it has been found that the compositions according to the present invention allow the formulation of stable and sprayable sun care o/w emulsions, the interest of which lies in their capacity to invert very quickly into a w/o emulsion while rubbing on the skin. The formation of a lipophylic film on the skin surface is much faster than the normal inversion process that takes place when a standard o/w emulsion is applied and the water phase evaporates. The sprayable emulsion permits to claim a water resistance effect without including any waterproof ingredients. This allows boosting the long lasting SPF protection without wash-off while bathing. The experiments supporting the present invention also conclude the ability of the compositions to reduce TEWL showing long-term moisturising effect of epidermis Therefore, this specific emulsifying system is applicable in all those applications where a high skin protection is required (for example body lotion for dry skin, care lotion for babies and children). Finally, and endorsed by its positive sensory profile (mainly in terms of absorption, tackiness and smoothness), this in-situ w/o emulsion generates the sensory characteristics of an o/w emulsion with the benefits of a w/o one.

### Oil components

Suitable oil components (component a) are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C ₁₃-carboxylic acids with linear or branched C₆-C ₂₂-fatty alcohols, such as, for example, propylheptyl caprylate, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C ₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.), aliphatic or naphthenic hydrocarbons, such as, for example, squalane, squalene or dialkylcyclohexanes, and/or mineral oils. The preferred oil components, which give the highest benefit from an applicational point of view, can be chosen from the group consisting of tri- or partial glycerides, dialkyl ethers, dialkyl carbonates.

### W/O Emulsifiers

Typically, the w/o emulsifiers (component b) are chosen from the group consisting of sorbitan esters and polyglycerol esters:

### Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 mol, and preferably 5 to 10 mol, ethylene oxide onto the sorbitan esters mentioned are also suitable.

### Polyglycerol esters

Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan® PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor® GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

### Cationic surfactants

Cationic surfactants (Component c) may be chosen from the group of tetraalkylammonium salts, like for example distearyldimethylammonium chloride (DSDMAC), cetrimonium chloride or behentrimonium chloride. The preferred compounds, however, represent esterquats.

### Esterquats

"Esterquats" are generally understood to be quaternised fatty acid triethanolamine ester salts. These are known compounds which can be obtained by the relevant methods of preparative organic chemistry. Reference is made in this connection to International patent application WO 91/01295 (Henkel), according to which triethanolamine is partly esterified with fatty acids in the presence of hypophosphorous acid, air is passed through the reaction mixture and the whole is then quaternised with dimethyl sulphate or ethylene oxide. In addition, German patent DE 4308794 C1 (Henkel) describes a process for the production of solid esterquats in which the quaternisation of triethanolamine esters is carried out in the presence of suitable dispersants, preferably fatty alcohols. Typical examples of esterquats suitable for use in accordance with the invention are products of which the acyl component derives from monocarboxylic acids corresponding to formula **(I):**

**R¹CO-OH** (I)

in which R¹CO is an acyl group containing 6 to 10 carbon atoms. Examples of such monocarboxylic acids are caproic acid, caprylic acid, capric acid and technical mixtures thereof such as, for example, so-called head-fractionated fatty acid. Esterquats, of which the acyl component derives from monocarboxylic acids of formula **(I)**, in which R¹CO is a linear, saturated acyl group containing 8 to 10 carbon atoms, are preferably used.

Other esterquats used in accordance with the invention are those of which the acyl component derives from dicarboxylic acids corresponding to formula **(II)**:

**HOOC(CH₂)ₙCOOH** (II)

in which n is a number of 1 to 10. Examples of such dicarboxylic acids are malonic acid, succinic acid, maleic acid, fumaric acid, glutaric acid, sorbic acid, pimelic acid, azelaic acid, sebacic acid and/or dodecanedioic acid, but preferably adipic acid. Overall, esterquats of which the acyl component derives from a mixture of (a) monocarboxylic acids corresponding to formula **(I)**, where R¹CO is a linear saturated acyl group containing 6 to 22 carbon atoms, and of (b) adipic acid are preferably used. The molar ratio of acyl moiety (a) to acyl moiety (b) may be in the range from 1:99 to 99:1 and is preferably in the range from 50:50 to 90:10 and more particularly in the range from 70:30 to 80:20.

A special embodiment of the invention is characterized by the use of esterquats which represent quaternised fatty acid triethanolamine ester salts corresponding to formula **(III)**: 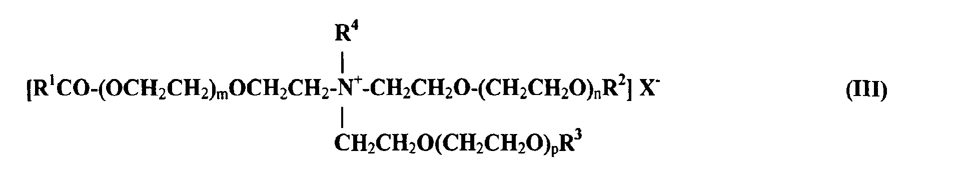 in which R¹CO stands for mono- and/or dicarboxylic acids, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulphate or alkyl phosphate.

Besides the quaternised fatty acid triethanolamine ester salts, other suitable esterquats are quaternised ester salts of mono- and/or dicarboxylic acids with diethanolalkylamines corresponding to formula **(IV)**: 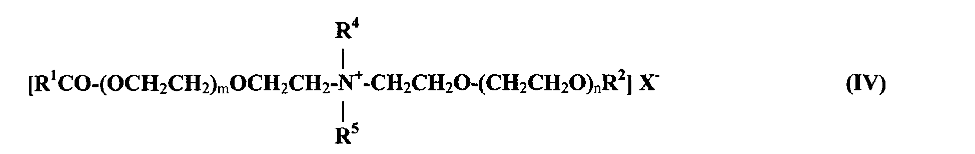 in which R¹CO represents a mono- and/or dicarboxylic acids R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulphate or alkyl phosphate.

Another group of suitable esterquats are the quaternised ester salts of mono- and/or dicarboxylic acid mixtures with 1,2-dihydroxypropyl dialkylamines corresponding to formula **(V)**: 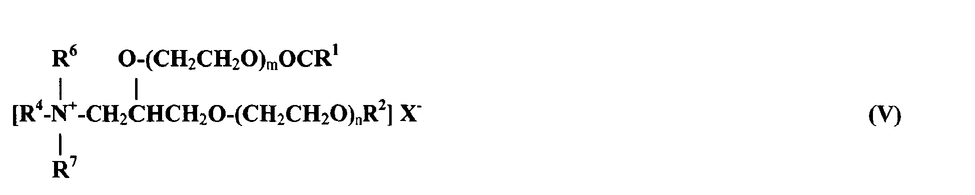 in which R¹CO represents a mono- and/or dicarboxylic acids, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulphate or alkyl phosphate.

In addition, other suitable esterquats are substances in which the ester bond is replaced by an amide bond and which - preferably based on diethylenetriamine - correspond to formula **(VI)**: 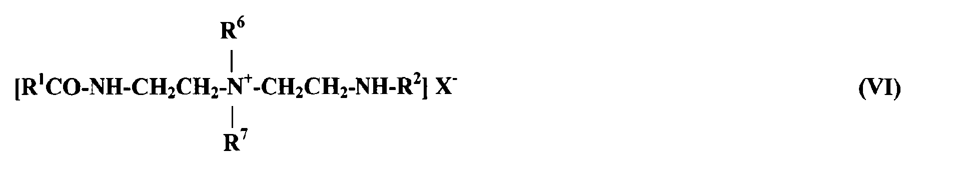 in which R¹CO represents a mono- and/or dicarboxylic acids, R² is hydrogen or has the same meaning as R¹CO, R⁶ and R⁷ independently of one another are alkyl groups containing 1 to 4 carbon atoms and X is halide, alkyl sulphate or alkyl phosphate. Amide esterquats such as these are commercially obtainable, for example, under the name of Incroquat^{®}. (Croda).

Finally, other suitable esterquats are compounds based on ethoxylated castor oil or hydrogenation products thereof which preferably correspond to formula **(VII)**: 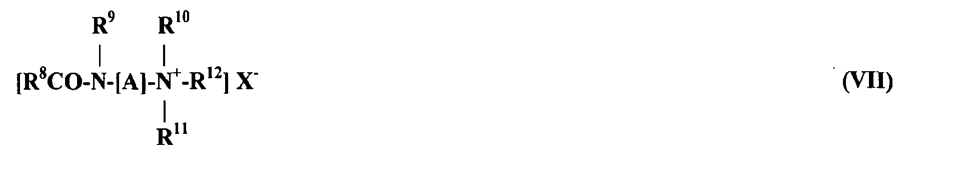 in which R⁸CO represents a mono- and/or dicarboxylic acids, A is a linear or branched alkylene group containing 1 to 6 carbon atoms, R⁹, R¹⁰ and R¹¹ independently of one another represent hydrogen or a C₁₋₄ alkyl group, R¹² is a C₁₋₄ alkyl group or a benzyl group and X is halogen, alkyl sulphate or alkyl phosphate. Such products are commercial available, for example, under the name of Demelan^{®} AU 39 BIO (Cognis):

So far as the choice of the preferred fatty acids and the optimal degree of esterification are concerned, the examples mentioned for **(III)** also apply to the esterquats corresponding to formulae **(IV)** to **(VII)**.

The esterquats corresponding to formulae **(III)** to **(VII)** may be obtained both from fatty acids and from the corresponding triglycerides in admixture with the corresponding dicarboxylic acids. One such process, which is intended to be representative of the relevant prior art, is proposed in European patent EP 0750606 B1 (Cognis). To produce the quaternised esters, the mixtures of mono- and dicarboxylic acids and the triethanolamine-based on the available carboxyl functions--may be used in a molar ratio of 1.1:1 to 3:1. With the performance properties of the esterquats in mind, a ratio of 1.2:1 to 2.2:1 and preferably 1.5:1 to 1.9:1 has proved to be particularly advantageous. The preferred esterquats are technical mixtures of mono-, di- and triesters with an average degree of esterification of 1.5 to 1.9. Particularly preferred are esterquats and esterquats compositions derived from fatty acid triethanolamine esters like Dehyquart^{®} L80, Dehyquart^{®} F 75, Dehyquart^{®} F100 or Dehyquart^{®} C 4046 which are described in more detail in EP 1119658 B1**,** EP 1128808 B1**,** EP 1131049 B1 and WO 00/027344 A1 (Cognis); as far as these products are concerned the teaching of the cited references is herewith fully incorporated by reference.

### Actives

The nature of the active ingredients (component d) is not critical since the selection fully depends on the required properties of the final product. Typically, the actives are selected from the group consisting of antioxidants, pigments, plant extracts and UV filters (primary and secondary sun protection factors), preferably liposoluble UV filters like, for example, chemicals filters and/or solutions of pigments, preferably TiO₂ in cosmetic oils, and their mixtures, which are explained in more detail in the following:

### Primary sun protection factors

Primary sun protection factors in the context of the invention are, for example, organic substances (light filters) which are liquid or crystalline at room temperature, and which are capable of absorbing ultraviolet radiation and of releasing the energy absorbed in the form of longer-wave radiation, for example heat. UV-B filters can be oil-soluble or water-soluble. The following are examples of oil-soluble substances:
o 3-benzylidene camphor or 3-benzylidene norcamphor and derivatives thereof, for example 3-(4-methylbenzylidene)-camphor;
o 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
o esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
o esters of salicylic acid, preferably salicylic acid-2-ethylhexyl ester, salicylic acid-4-isopropylbenzyl ester, salicylic acid homomenthyl ester;
o derivatives of benzophenone, preferably 2-hydroxy-4-methoxybenzophenone, 2-hydroxy-4-methoxy-4'-methylbenzophenone, 2,2'-dihydroxy-4-methoxybenzophenone;
o esters of benzalmalonic acid, preferably 4-methoxybenzalmalonic acid di-2-ethylhexyl ester;
o triazine derivatives such as, for example, 2,4,6-trianilino-(p-carbo-2'-ethyl-1'-hexyl-oxy)-1,3,5-triazine and Octyl Triazone or Dioctyl Butamido Triazone (Uvasorb^{®} HEB);
o propane-1,3-diones such as, for example, 1-(4-tert.butylphenyl)-3-(4'-methoxyphenyl)-propane-1,3-dione;
o ketotricyclo(5.2.1.0)decane derivatives;
o methylene bis-benzatriazolyltetramethylbutylphenol.

Suitable water-soluble substances are:
o 2-phenylbenzimidazole-5-sulfonic acid and alkali metal, alkaline earth metal, ammonium, alkylammonium, alkanolammonium and glucammonium salts thereof;
o sulfonic acid derivatives of benzophenones, preferably 2-hydroxy-4-methoxybenzophenone-5-sulfonic acid and salts thereof;
o sulfonic acid derivatives of 3-benzylidene camphor such as, for example, 4-(2-oxo-3-bomylidenemethyl)-benzene sulfonic acid and 2-methyl-5-(2-oxo-3-bomylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-meth-oxydi-benzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in oils, water or ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Cationic compatible stabilisers

Examples for suitable stabilisers (component d), compatible with the cationic surfactants forming component (c), are chose from the polyquaternium group. The following products have been found particularly useful:
o Polyquaternium-4, e.g. Celquat^{®} H100
o Polyquaternium-32 (and) Paraffinum liquidum, e.g. Rheocare^{®} CTC
o Polyquaternium-32 (and) Mineral Oil, e.g. Salcare^{®} SC 92
o Polyquatemium-37, e.g. Ultragel^{®} 300 (Cognis), Synthalen^{®} CN, Synthalen^{®} CR, Synthalen^{®} CU;
o Polyquaternium-37 (and) Propylene Glycol Dicaprylate/caprate (and) PPG-1 Trideceth-8, e.g. Cosmedia^{®} CTHE (Cognis), Salcare^{®} SC 96.

Also useful are Hydroxyethyl Cellulose (e.g. Natrosol^{®} 330 S Plus) and Hydroxypropyl Starch Phosphate (e.g. Structure XL).

### Meta-stable o/w emulsions

In a preferred embodiment of the present invention, the meta-stable o/w emulsions show the following composition:
(a) 10 to 35, preferably 10 to 30 % b.w. oil components,
(b) 1 to 7, preferably 2 to 5 % b.w. w/o emulsifiers,
(c) 0.1 to 5, preferably 0.5 to 3.5 % b.w. cationic surfactants,
(d) 0.1 to 35, preferably 0.5 to 20 % b.w. actives,
(e) 0.05 to 5, preferably 0.5 to 3 % b.w. cationic compatible stabilisers
on condition that the components add up to 100 % b.w with water and optionally other typical cosmetic ingredients. The emulsions according to the present invention show a viscosity according to Brookfield of up to 3,000 Cps (20 °C, spindle 5, 10 rpm) which allows them to be applied as a spray. Nevertheless, by adding thickeners it is possible to obtain also creams or lotions showing similar switching behaviour.

### Manufacturing process

Another object of the present invention is a first process for the preparation of aqueous meta-stable o/w emulsions with the ability to invert into w/o emulsions by introduction of mechanical energy, obtainable by the following method:
(a1) oil components, w/o emulsifiers and optionally oil-soluble actives are mixed at elevated temperatures to form an oil phase I,
(a2) cationic surfactants are diluted in water either at room temperature or at elevated temperatures to form an aqueous phase and this phase is maintained at elevated temperature (II)
(a3) phase (I) is added to phase (II) while maintaining the elevated temperature in order to obtain an o/w emulsion,
(a4) said o/w emulsion is cooled down to room temperature,
(a5) cationic compatible stabilisers and optionally water soluble actives are added, and
(a6) the final emulsion is homogenised.

In another preferred embodiment of the present invention the cationic compatible stabiliser is present during the emulsification process. In this case another object of the invention concerns a second process for the preparation of aqueous meta-stable o/w emulsions with the ability to invert into w/o emulsions by introduction of mechanical energy, obtainable by the following method:
(b1) oil components, w/o emulsifiers and optionally oil-soluble actives and/or cationic compatible stabilisers are mixed at elevated temperatures to form an oil phase I,
(b2) cationic surfactants and optionally cationic compatible stabilisers are diluted in water either at room temperature or at elevated temperatures to form an aqueous phase (II),
(b3) phase (I) is added to phase (II) while maintaining the elevated temperature in order to obtain an o/w emulsion,
(b4) said o/w emulsion is cooled down to room temperature,
(b5) optionally water soluble actives are added, and
(b6) the final emulsion is homogenised
on condition that said polymeric stabilisers are present in the oil or aqueous phase or both. The phrase "elevated temperatures" is to be understood as a temperature in the range of from about 50 to about 90, and preferably of from about 60 to about 80 °C.

In the alternative the emulsions are also obtainable by the following method:
(c1) oil components, w/o emulsifiers, cationic surfactant and/or compatible stabilizers and optionally oil-soluble actives are mixed at elevated temperatures to form an oil phase I,
(c2) optionally cationic compatible stabilisers are diluted in water either at room temperature or at elevated temperatures to form an aqueous phase (II),
(c3) phase (I) is added to phase (II) while maintaining the elevated temperature in order to obtain an o/w emulsion,
(c4) said o/w emulsion is cooled down to room temperature,
(c5) optionally water soluble actives are added, and
(c6) the final emulsion is homogenised.

### Industrial application

A final embodiment of the present invention refers to the use of said meta-stable o/w emulsions for making cosmetic and/or pharmaceutical compositions, in particular for making skin care compositions, or hair care compositions, or (water-resistant) sun care compositions, which may be present in the form of a lotion or a spray. For each specific purpose, the final products may comprise additional ingredients, like for example additional surfactants, additional oil bodies, additional emulsifiers, superfatting agents, pearlizing waxes, consistency factors, polymers, silicone compounds, waxes, stabilizers, hydrotropes, preservatives, solubilizers, perfume oils, dyes and the like.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 carbon atoms, preferably 16 to 18 carbon atoms, and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlizing waxes

Suitable pearlizing waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as, for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
o glycerol;
o alkylene glycols such as, for example, ethylene glycol, diethylene glycol, propylene glycol, butylene glycol, hexylene glycol and polyethylene glycols with an average molecular weight of 100 to 1000 Dalton;
o technical oligoglycerol mixtures with a degree of self-condensation of 1.5 to 10, such as for example technical diglycerol mixtures with a diglycerol content of 40 to 50% by weight;
o methylol compounds such as, in particular, trimethylol ethane, trimethylol propane, trimethylol butane, pentaerythritol and dipentaerythritol;
o lower alkyl glucosides, particularly those containing 1 to 8 carbon atoms in the alkyl group, for example methyl and butyl glucoside;
o sugar alcohols containing 5 to 12 carbon atoms, for example sorbitol or mannitol,
o sugars containing 5 to 12 carbon atoms, for example glucose or sucrose;
o amino sugars, for example glucamine;
o dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulfate, sodium thiosulfate and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Firbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of from 0.001 to 0.1 % by weight, based on the mixture as a whole.

The total percentage content of auxiliaries and additives may be from 1 to 50% by weight and is preferably from 5 to 40% by weight, based on the particular composition.

### Examples

### Examples 1 to 10

**Meta-stable o/w emulsions (amounts calculated as % b.w.)**

| **Phase** | **Compound** | **1** | **2** | **3** | **4** | **5** | **6** | **7** | **8** | **9** | **10** | **11** | **12** |
|---|---|---|---|---|---|---|---|---|---|---|---|---|---|
| **A** | **Natrosol^{®} 330 S Plus** Hydroxyethyl cellulose | - | - | - | - | 0.4 | - | - | - | - | - | - | - |
| | **Ultragel^{®} 300** Polyquatemium-37 | 0.6 | 0.6 | 0.6 | - | - | - | 0.6 | 0.6 | 0.6 | 1.0 | 1.0 | 0.6 |
| | **Cosmedia^{®} CTH (E)** Polyquatemium-37 (and) Propylene Glycol Dicaprylate/caprate (and) PPG-1 Trideceth-8 | - | - | - | 0.7 | - | - | - | - | - | - | - | - |
| | **Structure XL** Hydroxypropyl Starch Phosphate | - | - | - | - | - | 3.0 | - | - | - | - | - | - |
| | **Water** | add to 100 | | | | | | | | | | | |
| **B** | **Dehyquart^{®} L80** Dicocoylethyl Hydroxyethylmonium Methosulfate (and) Propylene Glycol | 1.0 | - | - | 1.0 | 1.0 | 1.0 | 1.0 | 1.0 | 1.0 | 1.0 | - | - |
| | **Dehyquart^{®} A** Cetrimonium Chloride | - | 1.0 | - | - | - | - | - | - | - | - | - | - |
| | **Dehyquart^{®} C 304** Palmitamidopropyl Trimonium Methosulfate | - | - | 1.0 | - | - | - | - | - | - | - | - | - |
| | **Genamin^{®} KDMP** Behentrimonium Chloride | - | - | - | - | - | - | - | - | - | - | 1.5 | 1.5 |
| **C** | **Dehymuls^{®} PGPH** Polyglyceryl-2 Dipolyhydroxystearate | 4.0 | 4.0 | 4.0 | 4.0 | 4.0 | 4.0 | 4.0 | 4.0 | 4.0 | 1.5 | 1.5 | 4.0 |
| | **Cetiol^{®} CC** Dicaprylyl Carbonate | 9.0 | 9.0 | 9.0 | 9.0 | 9.0 | 9.0 | 9.0 | 9.0 | 9.0 | 5.0 | 5.0 | 9.0 |
| | **Myritol^{®} 331** Cocoglycerides **Cetiol^{®} B** Dibutyl Adipate | 6.0 | 6.0 | 6.0 | 6.0 | 6.0 | 6.0 | - | 6.0 | 6.0 | - | - | 6.0 |
| | | - | - | - | - | - | - | 4.0 | - | - | - | - | - |
| | **Cegesoft^{®} PS 6** Oleus | 3.0 | 3.0 | 3.0 | 3.0 | 3.0 | 3.0 | | 3.0 | 3.0 | - | - | 3.0 |
| | **DC 200** Dimethicone | 0.5 | 0.5 | .05 | 0.5 | 0.5 | 0.5 | 0.5 | 0.5 | 0.5 | - | - | 0.5 |
| | **Phenonip^{®}** Phenoxyethanol (and) Methylparaben (and) Ethylparaben (and) Butylparaben (and) Propylparaben (and) Isobutylparaben | 0.5 | 0.5 | .05 | 0.5 | 0.5 | 0.5 | 0.5 | 0.5 | 0.5 | - | - | 0.5 |
| | **Neo Heliopan^{®} 303** Octocrylene | - | - | - | - | - | - | 3.0 | - | - | - | - | - |
| | **Neo Heliopan^{®} AV** Ethylhexyl Methoxycinnamate | - | - | - | - | - | - | 5.0 | - | - | - | - | - |
| | **Neo Heliopan^{®} 357** Butyl Methoxydibenzoyl Methane | - | - | - | - | - | - | 2.0 | - | - | - | - | - |
| **D** | **Perfume** | 0.2 | | | | | | | | | | | |
| | **Tocopheryl Acetate** | - | - | - | - | - | - | - | - | 0.3 | - | - | - |
| | **Ethanol** | - | - | - | - | - | - | - | - | 5.0 | 5.0 | 5.0 | 1.0 |

The compositions according to Examples 1 to 12 were prepared according to the following procedure:
(i) oil components, w/o emulsifiers and optionally oil-soluble actives were mixed at elevated temperatures to form an oil phase I,
(ii) cationic surfactants and cationic compatible stabilisers were diluted in water at about 70 °C to form an aqueous phase (II),
(iii) phase (I) was added to phase (II) while maintaining the elevated temperature in order to obtain an o/w emulsion,
(iv) said o/w emulsion was cooled down to room temperature, and
(v) finally, the emulsion was homogenised.

### Application of the meta-stable o/w emulsions

This sprayable o/w emulsion according to Example 1 turns into a w/o emulsion very quickly during skin application. To characterize the effects of this emulsion after skin application, Trans Epidermal Water Loss (TEWL) tests and waterproofing efficacy with Sun Protection Factor (SPF) determination were performed. The o/w emulsion showed a final conductivity of about 2000 µS/cm (Conductimeter Crison, Model Basic 30, RT) and a viscosity according to Brookfield (RVT, 23 °C, Spindle 5, 10 rpm) of 2,000 mPas.

### 1. Trans Epidermal Water Loss (TEWL) test

This study had the primary objective to evidence the effect of several cosmetic formulations on TEWL, on volunteers after one single standardized application. The protocol consisted in applying 3µl/cm² of product (single standardized application) on the forearm of 11 volunteers aged from 18 to 46. TEWL was measured with a Tewameter^{®} at T0, T1h, T2h, T4h and T24h. A TEWL decrease characterizes a decrease of water exchanges from the epidermis to the external environment. This could either mean an improvement of the skin's barrier function or an occlusive effect of the product. However, when the TEWL decrease is still present 24h after application, the hypothesis of the skin's barrier function improvement is more probable. The results are shown in Table 2.

**Table 2**

| **Decrease in TEWL of cosmetic compositions [g*m^{-2*}h⁻¹]** | | | | | |
|---|---|---|---|---|---|
| **Time [h]** | **0** | **1** | **2** | **4** | **24** |
| Non-treated | 11.5 | 11.1 | 10.9 | 10.6 | 10.7 |
| Standard w/o emulsion | 11.7 | 11.5 | 10.9 | 9.9 | 9.7 |
| meta-stable o/w emulsion Example 1 | 11.4 | 9.3 | 9.1 | 91 | 9.1 |

Compared with a non-treated zone, the inventive meta-stable o/w emulsion induced a significant TEWL decrease one, two, four and twenty-four hours after a single standardized application (from -12% to -16% on average). Under the same application conditions, a standard w/o emulsion did not induce any significant TEWL variations one and two hours after application.

### 2. Determination of sun protection factor and water resistance

The Sun Protection Factor Determination of the tested emulsion according to Example 5 was determined according to the International Sun Protection Factor (SPF) Test Method, COLIPA (12 volunteers aged from 18-56, phototype I, II or III, UV source 300 W Xenon Solar Light). The procedure used for the water resistance test was the shower method with 2 min 30 sec in water at a temperature of 23°C. The water resistance criterion was SPF>50% of the SPF without immersion in the same subjects (10 volunteers). The results are shown in Table 3.

**Table 3**

| **SPF acc. to Colipa, 12 volunteers; Shower test: 23°C tap water 70°, 2min 30s** | | | | | | |
|---|---|---|---|---|---|---|
| **Product** | **SPF** (mean) | **SPF** (labelled) | **SD** | **95 % CI** | **PCD** | **Water Resistance** |
| Emulsion acc. Example 1 | *Before immersion* | | | | | |
| | 26.2 | 25.0 | 6.1 | 21.8-30,5 | high | |
| | *After immersion* | | | | | |
| | 15.1 | | 3.0 | 13.2-17.0 | | yes |

On the basis of the average SPF value of 26.2 determined, the recommended maximum-labelled SPF (Colipa Recommendation N°11) for the test product is a SPF 25. The result for the test product after water immersion evidences that this formulation complies with the water resistance criteria.

## Claims

1. Aqueous meta-stable o/w emulsions with the ability to invert into w/o emulsions by introduction of mechanical energy, comprising
(a) oil components,
(b) w/o emulsifiers,
(c) cationic surfactants
(d) actives, and
(e) cationic compatible stabilisers.

2. Emulsions according to Claim 1, **characterised in that** the oil components are selected from the group consisting of tri- or partial glycerides, dialkyl ethers, dialkyl carbonates, and their mixtures.

3. Emulsions according to Claims 1 and/or 2, **characterised in that** the w/o emulsifiers represent sorbitol esters and/or polyglycerol esters.

4. Emulsions according to any of Claims 1 to 3 **characterised in that** the cationic surfactants are selected from the group consisting of tetraalkylammonium salts and esterquats.

5. Emulsions according to any of Claims 1 to 4 **characterised in that** the actives are selected from the group consisting of antioxidants, pigments, plant extracts and UV filters.

6. Emulsions according to any of Claims 1 to 5, **characterised in that** the cationic compatible stabilisers are selected from the group consisting of Polyquaternium 4, Polyquaternium 32, Polyquaternium 37, Hydoxypropyl Cellulose and Hydroxypropyl Starch Phosphate.

7. Emulsions according to any of Claims 1 to 6, **characterised in that** they comprise
(a) 10 to 35 % b.w. oil components,
(b) 1 to 7 % b.w. w/o emulsifiers,
(c) 0.1 to 5 % b.w. cationic surfactants,
(d) 0.1 to 35 % b.w. actives, and
(e) 0.05 to 5 % b.w. cationic compatible stabilisers
on condition that the components add up to 100 % b.w. with water and optionally other typical cosmetic ingredients.

8. Emulsions according to any of Claims 1 to 7, **characterised in that** they show viscosity according to Brookfield of up to 3,000 Cps (20 °C, spindle 5, 10 rpm).

9. Process for the preparation of aqueous meta-stable o/w emulsions with the ability to invert into w/o emulsions by introduction of mechanical energy, obtainable by the following method:
(a1) oil components, w/o emulsifiers and optionally oil-soluble actives are mixed at elevated temperatures to form an oil phase I,
(a2) cationic surfactants are diluted in water either at room temperature or at elevated temperatures to form an aqueous phase and this phase is maintained at elevated temperature (II)
(a3) phase (I) is added to phase (II) while maintaining the elevated temperature in order to obtain an o/w emulsion,
(a4) said o/w emulsion is cooled down to room temperature,
(a5) cationic compatible stabilisers and optionally water soluble actives are added, and
(a6) the final emulsion is homogenised.

10. Process for the preparation of aqueous meta-stable o/w emulsions with the ability to invert into w/o emulsions by introduction of mechanical energy, obtainable by the following method:
(b1) oil components, w/o emulsifiers and optionally oil-soluble actives and/or cationic compatible stabilisers are mixed at elevated temperatures to form an oil phase I,
(b2) cationic surfactants and optionally cationic compatible stabilisers are diluted in water either at room temperature or at elevated temperatures to form an aqueous phase (II),
(b3) phase (I) is added to phase (II) while maintaining the elevated temperature in order to obtain an o/w emulsion,
(b4) said o/w emulsion is cooled down to room temperature,
(b5) optionally water soluble actives are added, and
(b6) the final emulsion is homogenised
on condition that said polymeric stabilisers are present in the oil or aqueous phase or both.

11. Process for the preparation of aqueous meta-stable o/w emulsions with the ability to invert into w/o emulsions by introduction of mechanical energy, obtainable by the following method:
(c1) oil components, w/o emulsifiers, cationic surfactant and/or compatible stabilizers and optionally oil-soluble actives are mixed at elevated temperatures to form an oil phase I,
(c2) optionally cationic compatible stabilisers are diluted in water either at room temperature or at elevated temperatures to form an aqueous phase (II),
(c3) phase (I) is added to phase (II) while maintaining the elevated temperature in order to obtain an o/w emulsion,
(c4) said o/w emulsion is cooled down to room temperature,
(c5) optionally water soluble actives are added, and
(c6) the final emulsion is homogenised.

12. Use of emulsions according to any of Claims 1 to 8 for making cosmetic and/or pharmaceutical compositions.

13. Use according to Claim 12, **characterised in that** said emulsion is a skin care composition, a hair care composition, or a sun care composition.

14. Use according to Claims 12 and/or 13, **characterised in that** said emulsion is a water-resistant sun care composition.

15. Use according to any of Claims 12 to 14, **characterised in that** said emulsion is a lotion or a spray.
